# EUROPÄISCHE PATENTSCHRIFT

(11) **EP 2 111 894 B1**
(45) Veröffentlichungstag und Bekanntmachung des Hinweises auf die Patenterteilung: **13.04.2011**
(21) Anmeldenummer: 09156055.7
(22) Anmeldetag: 24.03.2009
(51) Int. Cl.: A61N 1/37

(54) **Herzstimulator mit Stimulationserfolgskontrolle**
Heart stimulator with stimulation success control
Stimulateur cardiaque doté d'un contrôle de la réussite de la stimulation

(30) Priorität: 22.04.2008 DE 102008020124
(43) Veröffentlichungstag der Anmeldung: 28.10.2009
(73) Patentinhaber: Biotronik CRM Patent AG, 6341 Baar (CH)
(72) Erfinder: Dörr, Thomas, 12437, Berlin (DE); Tscherch, Frank, 15741, Bestensee (DE); Tietze, Ulrich, 13156, Berlin (DE)
(74) Vertreter: Lindner-Vogt, Karin L.

(56) Entgegenhaltungen:
- US-A1- 2006 287 681
- US-A1- 2006 287 685
- US-A1- 2007 078 489

## Beschreibung

Die Erfindung betrifft einen Herzstimulator mit Stimulationserfolgskontrolle, d. h. einen Herzstimulator, wie einen implantierbaren Herzschrittmacher oder einen implantierbaren Kardioverter/Defibrillator (ICD), der selbsttätig in der Lage ist, die Wirksamkeit eines an ein Herz abgegebenen Stimulationsimpulses zu erfassen.

Herzstimulatoren der vorgenannten Art sind insbesondere als implantierbare Herzschrittmacher bereits bekannt. Solche Herzschrittmacher sind im implantierten und funktionsfähigen Zustand üblicherweise über eine Elektrodenleitung der vorgenannten Art mit einer im Herzen angeordneten Elektrode verbunden und ausgebildet, über die Elektrode elektrische Stimulationsimpulse an das Herz - oder genauer: an einzelne Kammern eines Herzens - abzugeben. Diese Stimulationsimpulse dienen der Erregung des Herzgewebes (Myokards) der jeweiligen Kammer und werden je nach Herzschrittmacherart insbesondere dann abgegeben, wenn sich das Herz nicht zur rechten Zeit auf natürliche Weise kontrahiert. Eine Kontraktion wird dann durch einen elektrischen Impuls hervorgerufen, der an das Myokard abgegeben wird. Dieser elektrische Impuls hat - wenn er ausreichend bemessen ist - die Wirkung, dass das Herzmuskelgewebe örtlich depolarisiert wird und entsprechend kontrahiert. Die Depolarisation und Kontraktion des Herzmuskelgewebes soll sich über den gesamten stimulierten Herzmuskel ausdehnen und so zur gewünschten Kontraktion der entsprechenden Herzkammer führen.

Der entsprechende elektrische Stimulationsimpuls muss eine Stimulationsintensität besitzen, die über einer jeweiligen Reizschwelle des Herzmuskelgewebes liegt. Die Reizschwelle ist dabei ein Maß für die Mindeststimulationsintensität, die ausreicht, um eine Depolarisation des Myokards und damit eine Kontraktion einer jeweiligen Kammer des Herzens zu bewirken. Die Reizschwelle hängt von verschiedenen Faktoren ab und ist darüber hinaus im Laufe der Zeit unter Umständen auch veränderlich. Neben dem Erfordernis, einen Stimulationsimpuls ausreichender Stimulationsintensität abzugeben, besteht das Bedürfnis, die für einen Stimulationsimpuls aufzuwendende Energie so gering wie möglich zu halten. Diese Energie wird regelmäßig einer Batterie des Herzschrittmachers entnommen, die sich im Laufe der Zeit erschöpft. Wenn diese Batterie erschöpft ist, muss ein implantierter Herzschrittmacher (in einer Operation) durch einen neuen Herzschrittmacher ersetzt werden. Eine möglichst lange Betriebsdauer des Herzschrittmachers und damit eine möglichst lange Batterielebensdauer ist somit wünschenswert. Die Energie für einen Stimulationsimpuls soll außerdem auch deshalb möglichst gering, aber ausreichend sein, um nur das Myokard, aber nicht umliegendes Muskelgewebe zu stimulieren.

Es besteht somit einerseits das Erfordernis, dass die Stimulationsintensität eines Stimulationsimpulses ausreichen muss, eine Kontraktion des Herzmuskelgewebes auszulösen. Eine höhere Stimulationsintensität geht bei im Übrigen unveränderten Einflussgrößen mit einem größeren Energieverbrauch einher. Die Stimulationsintensität hängt zum einen von der Dauer eines Stimulationsimpulses und andererseits von der Stärke eines Stimulationsimpulses ab. Die Stärke eines Stimulationsimpulses wiederum hängt von der elektrischen Spannung ab, mit der ein Stimulationsimpuls an das Herzmuskelgewebe abgegeben wird. Daher führt eine höhere Stimulationsintensität regelmäßig auch zu einem größeren Energieverbrauch. Um eine sichere Stimulation des Herzmuskelgewebes zu erreichen, werden regelmäßig Stimulationsimpulse abgegeben, die in energetischer Hinsicht etwas mehr Energie kosten, als minimal erforderlich wäre.

Andererseits besteht das Bedürfnis, den Energieverbrauch pro Stimulationsimpuls so gering wie möglich zu halten, da diese Energie einer Batterie des Herzschrittmachers entnommen wird, die auf diese Weise erschöpft wird.

Aus diesem Grunde sind Herzstimulatoren mit einer Stimulationserfolgskontrolle bekannt. Dabei kann die Stimulationserfolgskontrolle nach jeder Abgabe eines Stimulationsimpulses erfolgen (Beat-to-Beat), um im Falle eines ineffektiven Stimulus einen stärkeren nachzureichen (Automatic Capture Control ACC). Oder die Stimulationserfolgskontrolle erfolgt im Rahmen eines automatischen Reizschwellentests (Automatic Threshold Test, ATT), bei dem gezielt Stimulationsimpulse unterschiedlicher Intensität ausgelöst werden, um so zu ermitteln, ab welcher Intensität ein Stimulationsimpuls wirksam ist und damit die jeweilige Reizschwelle zu bestimmen.

In allen Fällen kommt es auf eine möglichst zuverlässige Erfassung eines Stimulationserfolgs - also einer stimulierten Kontraktion des jeweiligen Herzmuskelgewebes - an.

In diesem Zusammenhang ist es bekannt, dass die Messung der Reizschwelle (Nachweis einer effektiven Stimulation) an der Stimulationselektrode erfolgt, dadurch bedingt sind Messunsicherheiten durch Polarisations-, Autoshort- und Blanking Artefakte.

Auch sind nicht alle Elektrodentypen für die Methode geeignet, das bekannte Verfahren ist deshalb nicht uneingeschränkt nutzbar.

Die Erfindung besteht in einem implantierbaren Herzschrittmacher gemäß Anspruch 1.

Dokument US-A-2006/287681 offenbart einen Herzschrittmacher gemäß Präambel des Anspruchs 1.

Folgende Ausführungsvarianten der Stimulationserfolgserfassungseinheit sind hinsichtlich der Bewertung des Stimulationserfolgs durch die Stimulationserfolgserfassungseinheit bevorzugt:
- die Bewertung des Stimulationserfolgs beruht auf der Auswertung des zeitlichen Abstandes zwischen einem Stimulationsimpuls und der im Fernfeldelektrogramm aufgezeichneter Ventrikelerregung
- die Bewertung des Stimulationserfolgs beruht auf der Auswertung der dominierenden Signalpolarität der im Fernfeldelektrogramm aufgezeichneten Ventrikelerregung
- die Bewertung des Stimulationserfolgs beruht auf der Auswertung des zeitlichen Abstandes zwischen einem Stimulationsimpuls und der im Fernfeldelektrogramm aufgezeichneten Ventrikelerregung und deren dominierender Signalpolarität
- die Bewertung des Stimulationserfolgs beruht auf dem Vergleich eines Satzes von Parametern, die aus dem der Stimulation folgenden Fernfeldelektrogramm bestimmt werden, mit einem Musterparametersatz. Dieser repräsentiert entweder für Capture oder für Non-Capture. Es können auch zwei Musterparametersätze zur Verfügung stehen, so dass die Stimulationserfolgserfassungseinheit eine Art Gegenprobe durch doppelten Vergleich durchführen kann.

Gemäß einer weiteren bevorzugten Ausführungsvariante des Herzstimulators wird die gemessene Reizschwelle als diagnostische Information gespeichert und per Home Monitoring auf ein externes Gerät übertragen. Dazu weist der Herzstimulator einen entsprechenden Speicher und eine Telemetrieeinheit auf.

Im Sinne der zuvor geschilderten Varianten kann der Herzstimulator (oder genauer: dessen Stimulationssteuereinheit) ausgebildet sein, auf Basis der gemessenen Reizschwelle eine automatische Anpassung der Stimulationsenergie im Sine eines Automatic Threshold Test (ATT), für eine Dauer bis zum nächsten Test, also nicht Beat to Beat, durchführen.

Alternativ kann der Herzstimulator bzw. dessen Stimulationssteuereinheit ausgebildet sein, auf Basis der gemessenen Reizschwelle eine Beat to Beat Anpassung der Stimulationsimpulsintensität im Sinne einer Automatic Capture Control (ACC) vorzunehmen. Hierbei ist es vorteilhaft, wenn der Herzstimulator ausgebildet ist, im Falle einer Unterschreitung der Reizschwelle (also bei Detektion eines mangelnden Stimulationserfolges) einen möglichst effektiven Stimulus auszulösen.

Die Erfindung soll nun anhand eines Ausführungsbeispiels mit Bezug auf die Figuren näher erläutert werden. Von diesen zeigt
- Figur 1:: einen implantierbaren 3-Kammer Herzstimulator mit 3 Elektroden zum Her- zen des Patienten
- Figur 2:: ein Blockschaltung zur Realisierung der Reizschwellenmessfunktion; und
- Figur 3:: einen Algorithmus zur Feststellung der Effizienz der ventrikulären Stimula- tion.

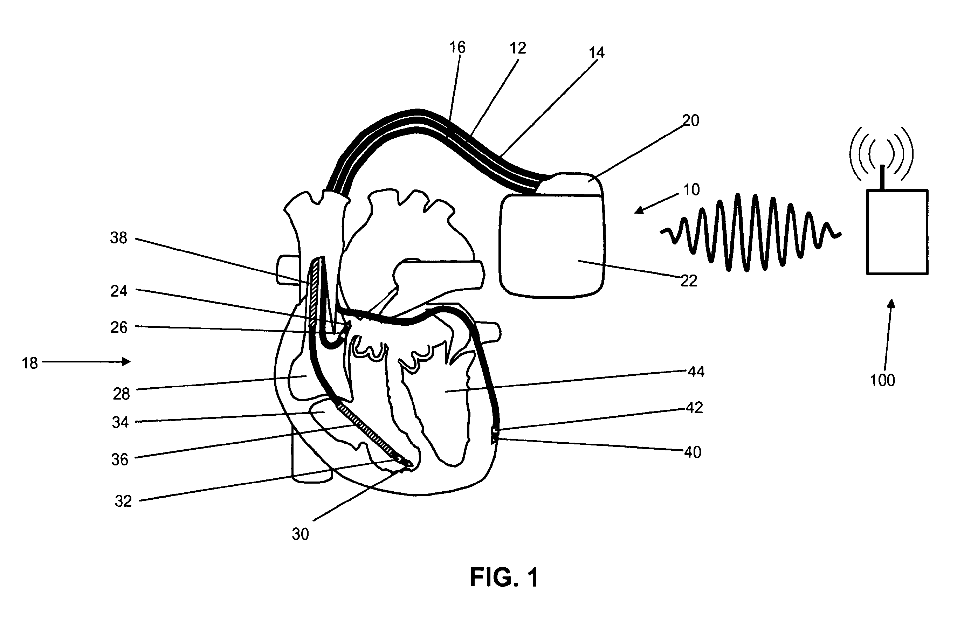

Figur 1 zeigt einen implantierbaren Herzstimulator 10 in Form eines Dreikammerherzschrittmachers/Kardioverter/Defibrillators mit daran angeschlossenen Elektrodenleitungen 12, 14 und 16 in Verbindung mit einem Herz 18. Außerdem ist ein externes Gerät 100 in der Nähe des implantierbaren Herzstimulators 10 dargestellt. Die Elektrodenleitungen 12, 14 und 16 sind über bekannte, standardisierte Steckverbindungen mit Kontaktbuchsen in einem Header (Anschlussgehäuse) 20 des Herzstimulators 10 elektrisch verbunden. Auf diese Weise sind die Elektrodenleitungen 12, 14 und 16 auch mit elektronischen Komponenten im Inneren eines hermetisch dichten Metallgehäuses 22 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 12 ist eine rechtsatriale Elektrodenleitung und besitzt an ihrem distalen Ende eine atriale Spitzenelektrode RA Tip 24 sowie in kurzer Entfernung davon eine atriale Ringelektrode RA Ring 26, die beide im rechten Atrium 28 des Herzens 18 platziert sind.

Die Elektrodenleitung 14 ist eine rechtsventrikuläre Elektrodenleitung und besitzt an ihrem distalen Ende eine rechtsventrikuläre Spitzenelektrode RV Tip 30 und in unmittelbarer Nähe eine rechtsventrikuläre Ringelektrode RV Ring 32. Beide Elektroden sind im Apex des rechten Ventrikels 34 des Herzens 18 angeordnet.

Außerdem trägt die rechtsventrikuläre Elektrodenleitung 14 noch eine rechtsventrikuläre Schockwendel RV Schock 36 als großflächige Elektrode zur Abgabe von Defibrillationsschocks. Eine weitere Schockwendel 38 ist in der Vena Cava Superior angeordnet und wird deshalb nachfolgend auch als SVC-Schockelektrode bezeichnet.

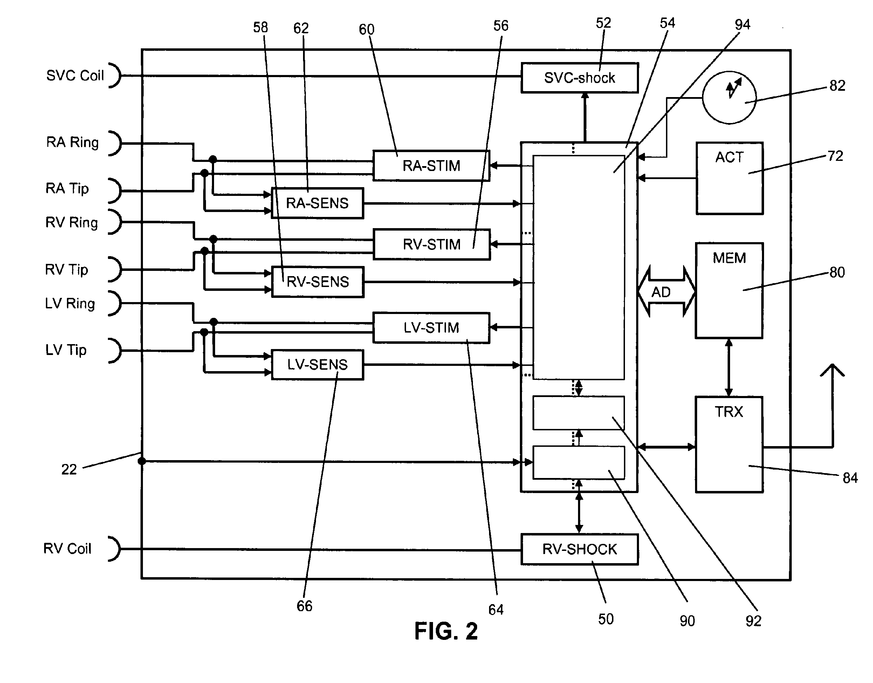

Die Elektrodenleitung 16 ist eine linksventrikuläre Elektrodenleitung, an deren distalem Ende eine linksventrikuläre Spitzenelektrode LV Tip 40 sowie in deren Nähe eine linksventrikuläre Ringelektrode LV Ring 42 angeordnet sind. Außerdem trägt die linksventrikuläre Elektrodenleitung 16 eine nicht näher bezeichnete, aber in Figur 2 dargestellte linksventrikuläre Schockwendel zur Abgabe von Defibrillationsschocks an den linken Ventrikel 44. Die linksventrikuläre Elektrodenleitung 16 wird vom rechten Atrium 28 des Herzens 18 aus über den Koronarsinus in eine von diesem abzweigende Lateralvene geführt und wird deswegen auch als Koronarsinuselektrodenleitung oder CS-Elektrodenleitung bezeichnet. Das Gehäuse 22 des Herzstimulators 10 ist wenigstens teilweise leitfähig und bildet selbst eine großflächige Elektrode zur Aufnahme von Fernfeldpotentialen des Myokards.

In Figur 2 sind die Hauptbestandteile des Herzstimulators 10 dargestellt. Auf der linken Seite sind die elektrischen Anschlüsse für die verschiedenen Elektroden 24, 26, 30, 32, 36, 38, 40 und 42 dargestellt. Die Schockelektroden 36 und 38 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode RV Ring die rechtsventrikuläre Spitzenelektrode RV Tip abzugeben. Alternativ ist es auch möglich, dass das Gehäuse 22 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 22 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 30. Diese Erregung breitet sich dann durch natürliche Reizleitung über den gesamten rechten Ventrikel 34 weiter aus und sorgt so für eine stimulierte Kontraktion des rechten Ventrikels 34.

Die rechtsventrikuläre Sensingeinheit 58 ist dazu ausgebildet, an dem Anschluss für die rechtsventrikuläre Ringelektrode RV Ring und die rechtsventrikuläre Tipelektrode RV Tip anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels 34 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 34, die durch Schwellwertvergleich detektiert werden kann. Die rechtsventrikuläre Sensingeinheit 58 gibt daraufhin ein entsprechendes, eine natürliche Kontraktion des rechten Ventrikels 34 anzeigendes Ausgangssignal an die Stimulationssteuereinheit 54 aus.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 28 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 28 kennzeichnet.

In gleicher Weise sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Der Herzstimulator 10 ist in der Lage, von allen Sonden EKG-Signale zu erfassen. Im Fall der rechtsventrikulären Elektrodenleitung 14, die mit Ihrer rechtsventrikulären Defibrillationselektrode (Schockwendel 36, RV-Coil) eine RV Schock-Sonde bildet, erfolgt die Ableitung des EKG's von dem Anschluss RV coil gegen das Gehäuse 22 des Implantats und stellt somit ein Fernfeldsignal (HV-EKG) dar. Die beiden Ableitungen der von der rechtsatrialen Elektrodenleitung 12 gebildeten atrialen Sonde und der von der linksventrikulären Elektrodenleitung 16 gebildeten LV Sonde sind bipolare Ableitungen und stellen ein Nahfeldsignal dar. Die rechtsventrikuläre Elektrodenleitung muss für die hier vorliegende Erfindung nicht notwendigerweise eine Stimulations-/Wahrnehmungseinrichtung besitzen, d. h. die rechtsventrikuläre Spitzenelektrode RV Tip 30 und die rechtsventrikuläre Ringelektrode RV Ring 32 sowie die Schockwendel 38 in der Vena Cava Superior können bei alternativen Ausführungsvarianten der Erfindung auch entfallen.

Die Steuereinheit 54 weist eine Fernfeldelektrogrammerfassungseinheit 90 auf, die einerseits mit dem Gehäuse 22 des Herzstimulators 10 und andererseits mit dem Anschluss RV-Coil für die rechtsventrikuläre Defibrillationselektrode 36 verbunden ist. Die Fernfeldelektrogrammerfassungseinheit 90 ist ausgebildet, auf Basis der an den beiden Eingängen im Betrieb anliegenden elektrischen Potentiale ein Fernfeldelektrokardiogramm zu ermitteln. Sie besitzt einen Ausgang, der mit einem Elektrogrammeingang einer Stimulationserfolgserfassungseinheit 92 verbunden ist und der Übertragung des jeweiligen Fernfeldelektrokardiogramms an die Stimulationserfolgserfassungseinheit 92 dient. Die Stimulationserfolgserfassungseinheit 92 besitzt außerdem noch einen Signaleingang, der mit einer Stimulationssteuereinheit 94 der Steuereinheit 54 verbunden ist und über den die Stimulationserfolgserfassungseinheit 92 Signale empfängt, die jeweils einen von der Kammerstimulationseinheit generierten und abgegeben Kammerstimulationsimpuls anzeigen.

Die Stimulationserfolgserfassungseinheit 92 ist ausgebildet, einen jeweils der Abgabe eines Kammerstimulationsimpulses zeitlich unmittelbar nachfolgenden Abschnitt eines empfangenen Fernfeldelektrogramms dahingehend auszuwerten, ob dieser eine wirksame oder eine unwirksame Kammerstimulation repräsentiert.

Die Stimulationssteuereinheit 94 ist mit der rechtsventrikulären Stimulationseinheit 56 und der linksventrikulären Stimulationseinheit verbunden und ausgebildet, diese bei Bedarf zur Erzeugung und Abgabe von Stimulationsimpulsen mit eingestellter Intensität anzusteuern. Weiter ist die Stimulationssteuereinheit 94 in der Lage, die Intensität der Stimulationsimpulse in Abhängigkeit des jeweils von der Stimulationserfolgserfassungseinheit bestimmten Stimulationserfolges einzustellen und nötigenfalls die Stimulationsimpulsintensität durch Einstellen der Dauer und/oder der Stärke (Amplitude) eines Stimulationsimpulses anzupassen.

Von den in Figur 2 im Blockschaltbild dargestellten Elementen gezeigt tragen die folgenden zur Realisierung einer Messfunktion für eine linksventrikuläre Stimulation im Sinne der Erfindung bei. Die rechtsatriale Stimulationseinheit und die rechtsatriale Sensingeinheit bilden einen rechten Vorhofkanal und dienen zur Synchronisierung der Abgabe von linksventrikulären Stimulationsimpulsen mit der Vorhofaktivität des Patienten. Eine Ausführung ohne diesen Vorhofkanal ist aber prinzipiell auch möglich. Die linksventrikuläre Stimulationseinheit ermöglicht die Abgabe von Schrittmacherimpulsen an die linke Herzkammer über eine über den Coronar Sinus verlegte linksventrikuläre Elektrodenleitung 16. Dabei werden die Impulsparameter wie Stimulationsamplitude und Impulsdauer durch die Stimulationseinheit vorgegeben.

Das EKG Signal, welches von der rechtsventrikulären Elektrodenleitung gegen das Gehäuse des Implantates abgeleitet wird, wird mittels eines EKG Verstärker-Blocks als Teil der Fernfeldelektrogrammerfassungseinheit 90 verstärkt und in geeigneter Weise breitbandig gefiltert. Durch die Stimulationssteuereinheit wird das so gewonnene Elektrogrammsignal bewertet und entschieden, ob ein über die linksventrikuläre Elektrodenleitung 16 abgegebener Stimulus effizient war oder nicht.

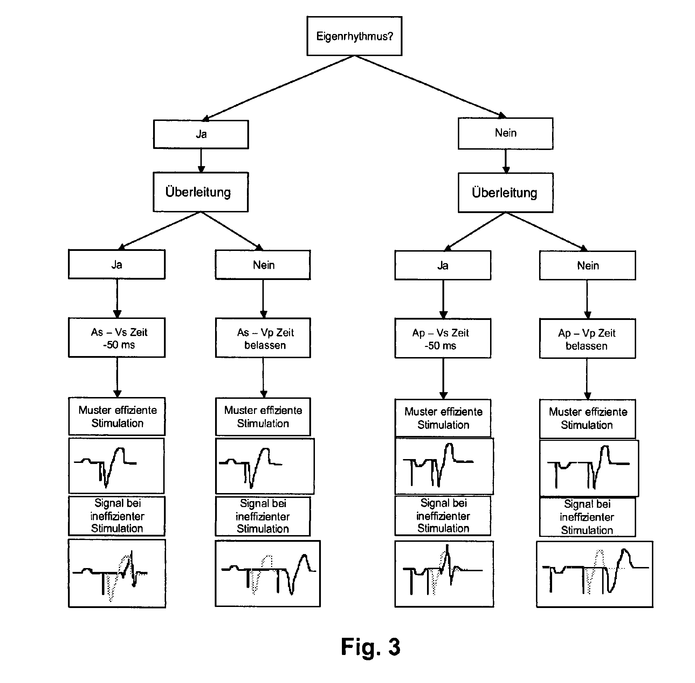

Anhand der Figur 3 wird ein möglicher Ablauf zur Feststellung der Effizienz von ventrikulären Stimulationen erläutert. Dabei spielt es prinzipiell keine Rolle, ob ein Stimulationsimpuls im linken oder im rechten Ventrikel abgegeben wird. Zunächst erfolgt die Feststellung des vorherrschenden Rhythmus bzw. Stimulationsmodus. Bei eigenem Sinusrhythmus werden die atrialen Stimulationsimpulse des Herzstimulators unterdrückt, bei fehlendem Sinusrhythmus erfolgt hingegen eine atriale Stimulation.

Wenn keine AV Blockierung vorliegt, wird der Ventrikel nicht stimuliert, bei Vorhandensein einer AV-Blockierung erfolgt eine Stimulation des Ventrikels. Entsprechend des gefundenen Modus wird bei sicherer ventrikulärer Stimulation ein Muster des dabei vorliegenden Fernfeldelektrokardiogramms aufgenommen. Anschließend kann für jede folgende ventrikuläre Stimulation die Effizienz durch einen möglichst einfachen Vergleich mit dem zuvor erfassten Muster geprüft werden. Bei nichteffizienter Stimulation kommt es dabei entweder zu einem kompletten Ausfall der ventrikulären Erregung oder zu einer zeitlichen Verschiebung. Bei Feststellung des Komplettausfalles (bei Vorliegen einer AV-Blockierung) erfolgt eine Stimulation mit sicherer Stimulationsamplitude und entsprechend verlängerter AV-Zeit.

## Patentansprüche

1. Implantierbarer Herzstimulator (10) mit
- einem wenigstens zum Teil elektrisch leitenden Gehäuse (22),
- einer Kammerstimulationseinheit (56; 64), die mit einer linksventrikulären oder einer rechtsventrikulären Stimulationselektrode verbunden oder zu verbinden und ausgebildet ist, Kammerstimulationsimpulse zur Stimulation eines Ventrikels eines Herzens zu generieren und abzugeben, und
- einem Anschluss für eine rechtsventrikuläre Defibrillationselektrode (36),
- einer Fernfeldelektrogrammerfassungseinheit (90) und
- einer Stimulationserfolgserfassungseinheit,
- von denen die Fernfeldelektrogrammerfassungseinheit (90) einen ersten Eingang aufweist, der mit dem Anschluss für die rechtsventrikuläre Defibrillationselektrode sowie einen zweiten Eingang, der mit dem elektrisch leitenden Gehäuse verbunden ist, und
- ausgebildet ist, auf Basis der an den beiden Eingängen im Betrieb anliegenden elektrischen Potentiale ein Fernfeldelektrokardiogramm zu ermitteln und an eine Stimulationserfolgserfassungseinheit auszugeben, und
- von denen die Stimulationserfolgserfassungseinheit (92) einen Elektrogrammeingang und einen Signaleingang aufweist und
- ausgebildet ist, über den Elektrogrammeingang ein von der Fernfeldelektrogrammerfassungseinheit (90) erzeugtes Fernfeldelektrogramm zu empfangen und über den Signaleingang eine Stimulationssignal zu empfangen, das einen von der Kammerstimulationseinheit (56, 64) generierten und abgegeben Kammerstimulationsimpuls anzeigt, und
- weiter ausgebildet ist, einen jeweils der Abgabe eines Kammerstimulationsimpulses zeitlich unmittelbar nachfolgenden Abschnitt eines empfangenen Fernfeldelektrogramms dahingehend auszuwerten, ob dieser eine wirksame oder eine unwirksame Kammerstimulation repräsentiert,
**dadurch gekennzeichnet dass**
der Herzstimulator eine Stimulationssteuereinheit (94) aufweist, die mit einem Steuerausgang der Stimulationserfolgserfassungseinheit (92) verbunden und die ausgebildet ist, die Abgabe von Kammerstimulationsimpulsen in Abhängigkeit davon, ob ein jeweils ausgewerteter Abschnitt eines empfangenen Fernfeldelektrogramms eine wirksame oder eine unwirksame Kammerstimulation repräsentiert, derart zu steuern, dass bei einer unwirksamen Kammerstimulation bei Vorliegen einer AV-Blockierung eine Stimulation mit sicherer Stimulationsamplitude und verlängerter AV-Zeit erfolgt.

2. Implantierbarer Herzstimulator nach Anspruch 1, **dadurch gekennzeichnet, dass** die Kammerstimulationseinheit eine rechtsventrikuläre Stimulationseinheit (56) ist.

3. Implantierbarer Herzstimulator nach Anspruch 1, **dadurch gekennzeichnet, dass** die Kammerstimulationseinheit eine linksventrikuläre Stimulationseinheit (64) ist.

4. Implantierbarer Herzstimulator nach einem der Ansprüche 1 bis 3, **dadurch gekennzeichnet, dass** die Stimulationserfolgserfassungseinheit (92) ausgebildet ist, eine Bewertung des Stimulationserfolgs durch Auswerten des zeitlichen Abstandes zwischen dem Zeitpunkt einer Stimulationsimpulsabgabe und der im Fernfeldelektrogramm aufgezeichneten Ventrikelerregung durchzuführen.

5. Implantierbarer Herzstimulator nach einem der Ansprüche 1 bis 3, **dadurch gekennzeichnet, dass** die Stimulationserfolgserfassungseinheit (92) ausgebildet ist, eine Bewertung des Stimulationserfolgs durch Auswerten der dominierenden Signalpolarität der im Fernfeldelektrogramm aufgezeichneten Ventrikelerregung durchzuführen.

6. Implantierbarer Herzstimulator nach einem der Ansprüche 1 bis 3, **dadurch gekennzeichnet, dass** die Stimulationserfolgserfassungseinheit (92) ausgebildet ist, eine Bewertung des Stimulationserfolgs durch Auswerten des zeitlichen Abstandes zwischen dem Zeitpunkt einer Stimulationsimpulsabgabe und der im Fernfeldelektrogramm aufgezeichneten Ventrikelerregung und der dominierenden Signalpolarität durchzuführen.

7. Implantierbarer Herzstimulator nach einem der Ansprüche 1 bis 3, **dadurch gekennzeichnet, dass** die Stimulationserfolgserfassungseinheit (92) ausgebildet ist, eine Bewertung des Stimulationserfolgs durch Vergleich eines Satzes von Parametern, bestimmt aus dem der Stimulation folgenden Fernfeldelektrogramm, mit einem Musterparametersatz durchzuführen.

8. Implantierbarer Herzstimulator nach Anspruch 7, **dadurch gekennzeichnet, dass** der Musterparametersatz einen Stimulationserfolg repräsentiert (Capture).

9. Implantierbarer Herzstimulator nach Anspruch 7, **dadurch gekennzeichnet, dass** der Musterparametersatz einen mangelnden Stimulationserfolg repräsentiert (Non-Capture).

10. Implantierbarer Herzstimulator nach einem der Ansprüche 1 bis 9, **dadurch gekennzeichnet, dass** der Herzstimulator (10) einen Speicher (80) und eine Telemetrieeinheit (84) aufweist und ausgebildet ist, eine gemessene Reizschwelle als diagnostische Information zu speichern und mittels der Telemetrieeinheit (84) drahtlos zu übertragen.

11. Implantierbarer Herzstimulator nach einem der Ansprüche 1 bis 10, **dadurch gekennzeichnet, dass** der Herzstimulator (10) ausgebildet ist, auf Basis einer gemessenen Reizschwelle eine automatische Anpassung der Stimulationsimpulsintensität vorzunehmen. (nicht Beat to Beat, d. h. ATT).

12. Implantierbarer Herzstimulator nach einem der Ansprüche 1 bis 10, **dadurch gekennzeichnet, dass** der Herzstimulator (10) ausgebildet ist, auf Basis einer gemessenen Reizschwelle eine Anpassung der Stimulationsenergie Impuls zu Impuls (Beat-to-Beat) vorzunehmen (ACC).

## Claims

1. An implantable cardiac stimulator (10) comprising
- an at least partially electrically conductive housing (22),
- a ventricular stimulation unit (56; 64) which is connected or will be connected to a left-ventricular or a right-ventricular stimulation electrode and is designed to generate and deliver ventricular stimulation pulses to stimulate a ventricle of a heart, and
- a connection for a right-ventricular defibrillation electrode (36),
- a far-field electrogram detection unit (90) and
- a stimulation success detection unit,
- of which the far-field electrogram detection unit (90) comprises a first input which is connected to the connection for the right-ventricular defibrillation electrode, and a second input which is connected to the electrically conductive housing, and
- is designed to ascertain a far-field electrocardiogram on the basis of the electrical potentials present at the two inputs during operation, and output it toa stimulation success detection unit, and
- of which the stimulation success detection unit (92) comprises an electrogram input and a signal input, and
- is designed to receive a far-field electrogram generated by the far-field electrogram detection unit (90) via the electrogram input, and, via the signal input, to receive a stimulation signal which indicates a ventricular stimulation pulse generated and delivered by the ventricular stimulation unit (58, 64), and
- is furthermore designed to evaluate a section of a far-field electrogram that was received and occurred at a time immediately after the delivery of a ventricular stimulation pulse to determine whether it represents an effective or an ineffective stimulation of the ventricle,
**characterized in that**
the cardiac stimulator comprises a stimulation control unit (94) which is connected to a control output of the stimulation success detection unit (92) and is designed to control the delivery of ventricular stimulation pulses as a function of whether a particular evaluated section of a received far-field electrogram represents an effective or an ineffective ventricular stimulation in a manner such that, if the ventricular stimulation was unsuccessful in the presence of an AV block, stimulation takes place using a reliable stimulation amplitude and an extended AV time.

2. The implantable cardiac stimulator according to claim 1, **characterized in that** the ventricular stimulation unit is a right-ventricular stimulation unit (56).

3. The implantable cardiac stimulator according to claim 1, **characterized in that** the ventricular stimulation unit is a left-ventricular stimulation unit (64).

4. The implantable cardiac stimulator according to one of the claims 1 to 3, **characterized in that** the stimulation success detection unit (92) is designed to evaluate the stimulation success by evaluating the time between the instant at which a stimulation pulse was delivered and the ventricular stimulation recorded in the far-field electrogram.

5. The implantable cardiac stimulator according to one of the claims 1 to 3, **characterized in that** the stimulation success detection unit (92) is designed to evaluate the stimulation success by evaluating the dominant signal polarity of the ventricular stimulation recorded in the far-field electrogram.

6. The implantable cardiac stimulator according to one of the claims 1 to 3, **characterized in that** the stimulation success detection unit (92) is designed to evaluate the stimulation success by evaluating the time between the instant at which a stimulation pulse was delivered and the ventricular stimulation recorded in the far-field electrogram, and the dominant signal polarity.

7. The implantable cardiac stimulator according to one of the claims 1 to 3, **characterized in that** the stimulation success detection unit (92) is designed to evaluate the stimulation success by comparing a set of parameters determined on the basis of the far-field electrogram recorded after the stimulation with a sample set of parameters.

8. The implantable cardiac stimulator according to claim 7, **characterized in that** the sample set of parameters represents stimulation success (capture).

9. The implantable cardiac stimulator according to claim 7, **characterized in that** the sample set of parameters represents the absence of stimulation success (non-capture).

10. The implantable cardiac stimulator according to one of the claims 1 to 9, **characterized in that** the cardiac stimulator (10) comprises a memory (80) and a telemetry unit (84) and is designed to store a stimulation threshold, which was measured, as diagnostic information and transmit it wirelessly using the telemetry unit (84).

11. The implantable cardiac stimulator according to one of the claims 1 to 10, **characterized in that** the cardiac stimulator (10) is designed to automatically adjust the stimulation pulse intensity on the basis of a stimulation threshold that was measured (not beat-to-beat i.e. ATT).

12. The implantable cardiac stimulator according to one of the claims 1 to 10, **characterized in that** the cardiac stimulator (10) is designed to adjust the stimulation energy beat-to-beat on the basis of a stimulation threshold that was measured (ACC).

## Revendications

1. Stimulateur cardiaque implantable (10), avec
- un boîtier (22) au moins partiellement électriquement conducteur,
- une unité de stimulation à chambres (56 ; 64), reliée ou destinée à être reliée à une électrode de stimulation ventriculaire gauche ou ventriculaire droite, conçue pour générer et émettre des impulsions de stimulation de chambre, pour stimuler un ventricule d'un coeur, et
- un raccord pour une électrode de défibrillation ventriculaire droite (36),
- une unité de détection d'électrogramme de champ lointain (90), et
- une unité de détection de stimulation réussie,
- parmi lesquelles l'unité de détection d'électrogramme de champ lointain (90) comporte une première entrée reliée au raccord pour l'électrode de défibrillation ventriculaire droite, ainsi qu'une deuxième entrée reliée au boîtier électriquement conducteur, et
- est conçue pour détecter un électrocardiogramme de champ lointain, sur la base des potentiels électriques appliqués aux deux entrées pendant le fonctionnement, et pour les livrer à une unité de détection de stimulation réussie, et
- parmi lesquelles l'unité de détection de stimulation réussie (92) comporte une entrée d'électrogramme et une entrée de signal, et
- est conçue pour recevoir, par le biais de l'entrée d'électrogramme, un électrogramme de champ lointain produit par l'unité de détection d'électrogramme de champ lointain, et pour recevoir, par le biais de l'entrée de signal, un signal de stimulation indiquant une impulsion de stimulation de chambre générée et délivrée par l'unité de stimulation à chambre (56, 64), et
- est en outre conçue pour évaluer une section d'un électrogramme de champ lointain reçu, qui est immédiatement consécutive dans le temps à la délivrance d'une impulsion de stimulation de chambre, pour vérifier si celui-ci représente une stimulation de chambre efficace ou inefficace,
**caractérisé en ce que**
le stimulateur cardiaque comporte une unité de commande de stimulation (94) reliée à une sortie de commande de l'unité de détection de stimulation réussie (92), et conçue pour commander la délivrance d'impulsions de stimulation de chambre, selon si une section évaluée d'un électrogramme de champ lointain représente une stimulation de chambre efficace ou inefficace, de manière à ce que lors d'une stimulation inefficace, en présence d'un blocage atrio-ventriculaire, une stimulation soit réalisée avec une amplitude de stimulation plus sûre et un temps atrio-ventriculaire prolongé.

2. Stimulateur cardiaque implantable selon la revendication 1, **caractérisé en ce que** l'unité de stimulation à chambre est une unité de stimulation ventriculaire droite (56).

3. Stimulateur cardiaque implantable selon la revendication 1, **caractérisé en ce que** l'unité de stimulation à chambre est une unité de stimulation ventriculaire gauche (64).

4. Stimulateur cardiaque implantable selon l'une des revendications 1 à 3, **caractérisé en ce que** l'unité de détection de stimulation réussie (92) est conçue pour évaluer la réussite de la stimulation, par l'évaluation du laps de temps entre la délivrance d'une impulsion de stimulation et l'excitation ventriculaire indiquée dans l'électrogramme de champ lointain.

5. Stimulateur cardiaque implantable selon l'une des revendications 1 à 3, **caractérisé en ce que** l'unité de détection de stimulation réussie (92) est conçue pour évaluer la réussite de la stimulation, par l'évaluation de la polarité de signal dominante de l'excitation ventriculaire indiquée dans l'électrogramme de champ lointain.

6. Stimulateur cardiaque implantable selon l'une des revendications 1 à 3, **caractérisé en ce que** l'unité de détection de stimulation réussie (92) est conçue pour évaluer la réussite de la stimulation, par l'évaluation du laps de temps entre la délivrance d'une impulsion de stimulation et l'excitation ventriculaire indiquée dans l'électrogramme de champ lointain et la polarité de signal dominante.

7. Stimulateur cardiaque implantable selon l'une des revendications 1 à 3, **caractérisé en ce que** l'unité de détection de stimulation réussie (92) est conçue pour évaluer la réussite de la stimulation, par la comparaison entre une série de paramètres, déterminée à partir de l'électrogramme de champ lointain suivant la stimulation, et une série de paramètres de consigne.

8. Stimulateur cardiaque implantable selon la revendication 7, **caractérisé en ce que** la série de paramètres de consigne représente une stimulation réussie (Capture).

9. Stimulateur cardiaque implantable selon la revendication 7, **caractérisé en ce que** la série de paramètres de consigne représente un échec de stimulation (Non-capture).

10. Stimulateur cardiaque implantable selon l'une des revendications 1 à 9, **caractérisé en ce que** le stimulateur cardiaque (10) comporte une mémoire (80) et une unité de télémétrie (84), et est conçu pour enregistrer un seuil de stimulation mesuré, en tant qu'information diagnostique, et pour transmettre celui-ci sans fil au moyen de l'unité de télémétrie (84).

11. Stimulateur cardiaque implantable selon l'une des revendications 1 à 10, **caractérisé en ce que** le stimulateur cardiaque (10) est conçu pour assurer une adaptation automatique de l'intensité d'impulsion de stimulation, sur la base d'un seuil de stimulation mesuré (pas Beat to Beat, donc ATT).

12. Stimulateur cardiaque implantable selon l'une des revendications 1 à 10, **caractérisé en ce que** le stimulateur cardiaque (10) est conçu pour assurer l'adaptation de l'énergie de stimulation impulsion par impulsion (Beat-to-Beat), sur la base d'un seuil de stimulation mesuré (ACC).
